(19) Europäisches Patentamt

European Patent Office

Office européen des brevets

(11) Veröffentlichungsnummer: **0 284 963 B1**

## EUROPÄISCHE PATENTSCHRIFT

(45) Veröffentlichungstag der Patentschrift: **07.08.91**

(51) Int. Cl.⁵: **G09F 3/02**

(21) Anmeldenummer: **88104579.3**

(22) Anmeldetag: **22.03.88**

(54) **Abziehhilfe sowie ihre Verwendung.**

(30) Priorität: **03.04.87 DE 3711256**

(43) Veröffentlichungstag der Anmeldung:
**05.10.88 Patentblatt 88/40**

(45) Bekanntmachung des Hinweises auf die
Patenterteilung:
**07.08.91 Patentblatt 91/32**

(84) Benannte Vertragsstaaten:
**AT BE CH DE ES FR GB GR IT LI LU NL SE**

(56) Entgegenhaltungen:
**EP-A- 0 102 599      DE-A- 2 407 822**
**US-A- 3 230 649      US-A- 3 361 252**
**US-A- 3 690 999      US-A- 4 335 172**

(73) Patentinhaber: **LTS Lohmann Therapie-**
**Systeme GmbH & Co. KG**
**Irlicherstrasse 55**
**W-5450 Neuwied 12(DE)**

(72) Erfinder: **Anhäuser, Dieter**
**Rengsdorfer Strasse 4**
**W-5451 Melsbach(DE)**

(74) Vertreter: **Neidl-Stippler, Cornelia, Dr.**
**Rauchstrasse 2**
**W-8000 München 80(DE)**

## Beschreibung

Die Erfindung betrifft eine Abziehhilfe für auf einem flächenförmigen flexiblen Trägermaterial haftende, mechanisch ablösbare Substrate in Form von Schnitten oder Sollbruchlinien im Trägermaterial sowie deren Verwendung.

Insbesondere bezieht sich die Erfindung auf die Trennung von zwei aneinanderhaftenden Materialien, von denen ein Material flächenförmig bzw. folienartig ist, die im Bereich einer Kontaktfläche übereinanderliegen, wie dies bspw. bei Pflastern oder Etiketten, die mit Schutzfolien für Haftklebeschichten ausgerüstet geliefert werden, der Fall ist.

Im folgenden werden die Flächen des Trägermaterials und des Substrats als Kontaktflächen bezeichnet; diese Flächen sind Teilabschnitte einer Trägermaterial- bzw. Substratoberfläche.

Ferner wird der Ausdruck "Substrat" sowohl für Substratabschnitte oder -teile als auch für das Substrat selbst verwendet.

Die vorliegende Erfindung bezieht sich demnach auf alle Kombinationen, die eine Substrat/Abdeckfolienkombination demzumindest als Unterkombination aufweisen.

Die Ablösung einer Abdeckmaterialschicht oder einer Abdeckfolie von einem bspw. mit einer Kleberschicht ausgerüsteten Substrat bereitet insbesondere dann Schwierigkeiten, wenn das Substrat weniger oder in etwa genauso flexibel wie die Abdeckfolie ist und ganz besonders dann, wenn die durch die Abdeckfolie bedeckte, nach Ablösen derselben ungeschützte Substratoberfläche vor Kontaminationen oder Beschädigungen während der Entfernung der Abziehfolie geschützt werden muß. Ein Grund für den Schutz der Substratkontaktfläche durch eine Abdeckfolie kann bspw. in der Aufrechterhaltung der Eigenklebrigkeit, deren Empfindlichkeit gegenüber mechanischer Beschädigung oder in einer Versiegelung derselben, bspw. gegen das Entweichen flüchtiger Komponenten des Substrats, liegen. Üblicherweise haftet das Trägermaterial, meist eine Folie, durch Adhäsionskräfte am Substrat, die durch Abziehen überwunden werden können.

Das Ablösen des Trägermaterials ist häufig problematisch und führt zu Verletzungen der zu schützenden Substratkontaktfläche, wenn bspw. mittels eines Fingernagels, eines Messers oder sonstiger Instrumente versucht wird, auf diesem verbleibende Trägermaterialteile vom Substrat zu entfernen. Dieses Verfahren versagt insbesondere dann, wenn das Verhältnis der Steifigkeit Substrat:Trägermaterial ungünstig ist, z.B. wenn das Trägermaterial sehr viel flexibler als das Substrat ist und auch leicht reißt oder wenn eine sehr weiche Substratkontaktfläche geschützt werden soll.

Insbesondere verbietet sich dieses Verfahren völlig, wenn eine Beeinträchtigung der Substratkontaktfläche durch Berührung - bspw. bei sterilen Flächen von Verbänden, den Steuerschichten von therapeutischen Pflastern oder reaktive Materialen aufweisenden Flächen vermieden werden muß.

Zur Lösung dieser Problematik ist bereits vorgeschlagen worden, geradlinige Schnitte oder Sollbruchlinien im Trägermaterial vorzusehen, wobei durch Abziehen oder Biegen des Trägermaterials Trägermaterialabschnitte vom Substrat abgelöst werden können.

Bisher konnte mittels dieser bekannten Lösungswege noch keine vollständige leichte Freilegung des Substrats, insbesondere dann, wenn es sich um ein relativ inflexibles, empfindliches Substrat - wie ein therapeutisches Pflaster oder bspw. ein Kunststofformteil und eine sehr flexible Trägerfolie, wie eine dünne Aluminiumfolie oder -laminat oder eine Polymerfolie, handelt, erzielt werden.

Insbesondere bei kleinen Substratflächen ist es schwierig, diese vollständig freizulegen.

Auch das selektive Abziehen einzelner Substratabschnitte von einem Trägermaterial ist nicht zufriedenstellend gelöst.

Das Vorsehen von gekrümmten Schnitten oder Sollbruchlinien in der Trägerfolie wurde in der US-PS 3 230 649 beschrieben und liefert bei Biegen des Materials die Bildung eines Anfaßabschnittes für einen Teil des Trägermaterials. Der verbleibende Rest muß jedoch noch mühsam mittels des Fingernagels oder eines Hilfswerkzeuges vom Substrat entfernt werden. Ein zufriedenstellendes Verfahren zum vollständigen unbeschädigten Ablösen des Substrats vom Trägermaterial ist dadurch nicht aufgezeigt. Bei mehreren Substratabschnitten auf demselben Trägermaterial kann auch eine Art Ablöseband die Oberflächen mehrerer Substratabschnitte miteinander verbinden, wobei durch Ziehen an einem überstehenden Teil des Bandes die vom Band verbundenen Substratabschnitte durch einen Arbeitsgang vom Trägermaterial abgezogen werden können. Vor Einsatz der Substratabschnitte müssen diese jedoch noch vom Ablöseband getrennt werden. Neben der relativ aufwendigen Herstellung dieser bekannten Anordung erlaubt sie nur das Ablösen vom mehreren Substratabschnitten gleichzeitig und ist auf kleindimensionierte Substratabschnitte beschränkt. Das Einlegen von Streifen oder Fäden zwischen Trägermaterial und Substrat, wobei die Streifen oder Fäden über den Rand der Substratabschnitte hinausragen, bietet ebenfalls eine Hilfe zum Ablösen der Substratabschnitte, ist aber mit einer aufwendigen Technologie verbunden.

Alle diese Vorschläge führen zu keiner zufriedenstellenden problemlosen Ablösung des Substrates vom Träger unter minimaler Beeinträchtigung des Substrats oder erfordern aufwendige Maßnahmen, wie das Vorsehen von Anfaßbändern.

Es ist daher Aufgabe der Erfindung, eine Abziehhilfe für Substrate vom Trägermaterial zu verwirklichen, die die Nachteile des Standes der Technik vermeidet.

Die Aufgabe wird bei einem gattungsgemäßen Gegenstand dadurch gelöst, daß mindestens in der Kontaktfläche des Trägermaterials für jedes Substrat eine gesonderte, nicht geradlinige Schnitt- oder Sollbruchlinie derart vorgesehen ist, daß bei Anwendung von Druck auf das Trägermaterial mit einer Kraftkomponente in Richtung senkrecht zur Substratkontaktfläche ein durch die Schnitt- oder Sollbruchlinie begrenzter Trägermaterialabschnitt mit auf ihm haftendem Substratabschnitt in Richtung des/der Substrats/e biegbar ist, wodurch mindestens ein Teil des Substrats in dessen Randbereich benachbart der Schnitt-oder Sollbruchlinie vom Trägermaterial abgelöst wird und damit ein Anfaßabschnitt am Substrat zum völligen Abziehen des Substrats gebildet wird.

Vorteilhafte Weiterbildungen des Erfindungsgedankens ergeben sich aus den Unteransprüchen.

Dadurch, daß erfindungsgemäß in jedem Trägermaterialkontaktabschnitt eine gesonderte nicht geradlinige Schnitt-oder Sollbruchlinie vorgesehen ist, ist bei Anwendung von Druck auf die freie Trägermaterialoberfläche im Bereich der Kontaktfläche ein Herausbiegen eines Teilabschnitts des Substrats aus der Kontaktflächenebene heraus möglich, wodurch ein Substrat-Anfaßabschnitt zum völligen Abziehen des Substrats abgelöst wird.

Hierdurch wird eine vollständige Ablösung des Substrats möglich.

Das Trägermaterial und/oder das Substrat können aus mehr als einer Schicht und verschiedenen Materialien aufgebaut sein, wobei bspw. dann, wenn das Substrat ein therapeutisches Pflaster ist, die Substratkontaktflächenschicht ggf. nicht durchgehend aus einheitlichem Material besteht.

Die dazu einsetzbaren Materialien müssen für das Trägermaterial flexibel und können für das Substrat auch starr sein. So eignen sich als Substrate auch Kunststofformkörper, wie "selbstklebende" Embleme, Wappen od. dgl.. Nach Abziehen des Substrats vom Trägermaterial wird eine Substratschichtoberfläche frei, die bspw. haftklebend ausgerüstet sein kann und/oder aber auch für Wirkstoffe, wie Arzneimittel, durchlässig ist - wie bspw. die Hautkontaktschicht von therapeutischen Pflastern, die nicht notwendigerweise haftklebend sein muß. In letzterem Falle erfüllt das Trägermaterial die Aufgabe einer Sperre, die das unerwünschte Herausdiffundieren von Wirkstoffen aus einem flächenförmigen therapeutischen System verhindert. Dabei ist es nicht unbedingt notwendig, daß die Kontaktfläche eben ist, sie kann auch entsprechend dem Einsatzzweck des Substrats geformt sein.

Die Erzeugung der Sollbruchlinien bzw. von Schnittlinien im Trägermaterial erfolgt nach an sich bekannten Verfahren, bspw. ist für die Herstellung von Schnitten Stanzen, Schneiden, Quetschen oder Prägen des Trägermaterials bevorzugt; es ist aber auch möglich, durch Laser oder Hochfrequenz zu schneiden. Sollbruchlinien können durch Anstanzen, Perforation, lokale chemische oder thermische Behandlung,insbesondere bei Polymerträgermaterialien, durch Laser-Schneiden od. dgl. erzeugt werden, wie es dem Fachmann auf diesem Gebiet geläufig ist. Selbstverständlich können die Linien vor oder nach Aufbringung des/der Substrats/Substrate hergestellt werden.

Nachfolgend wird die Erfindung anhand von Ausführungsbeispielen anhand der begleitenden Zeichnung näher erläutert, wobei letztere lediglich dem besseren Verständnis dienen und keinesfalls den Schutzbereich des Patentes einschränken sollen. Dabei zeigt:

Fig. 1   eine Aufsicht auf die Trägermaterialseite einer erfindungsgemäßen Substrat/Trägermaterialkombination mit runden Substraten

Fig. 2   einen Querschnitt durch die Kombination der Fig.1 entlang der Linie A-B,

Fig. 3   die Handhabung bei der Ablösung eines Substrats aus der Kombination gemäß Fig.1;

Fig. 4   die Ablösung eines Substratabschnitts, wie in Fig. 3 gezeigt, in einer Querschnittsansicht;

Fig. 5   eine Aufsicht auf eine weitere erfindungsgemäße Substrat/Trägermaterial-Kombination mit Schnitt- bzw. Sollbruchlinien gemäß der Erfindung mit verschiedenen Substraten unterschiedlicher geometrischer Gestalt;

Fig. 6   eine Aufsicht auf eine weitere erfindungsgemäße Substrat/Trägermaterial-Kombination mit oval-länglichen Substraten mit unterschiedlicher Schnittlinienführung;

Fig. 7   eine Aufsicht auf eine weitere erfindungsgemäße Substrat/Trägerkombination, bei der Trägermaterial und Substrat im wesentlichen die gleiche Fläche aufweisen; und

Fig. 8   eine Aufsicht auf eine Substrat/Trägerkombination mit großer Substrat/Trägermaterialkontaktfläche.

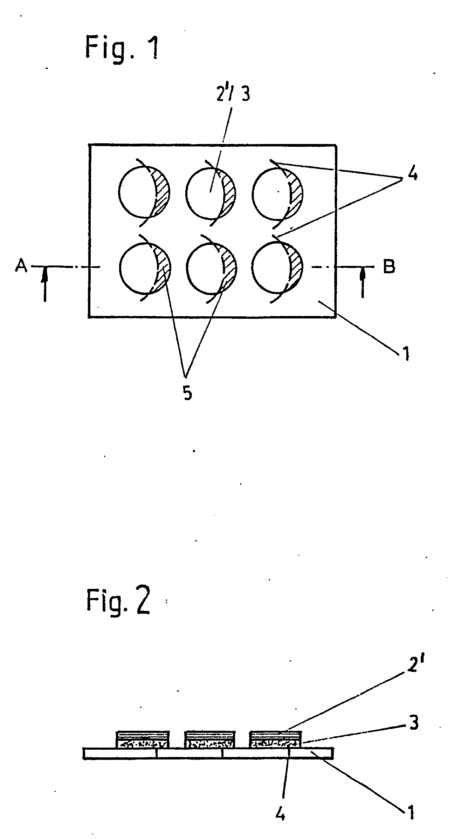

In Fig. 1 ist eine Ausführungsform einer erfindungsgemäßen Kombination aus Substrat 2 und Trägermaterial 1 schematisch dargestellt, wobei auf einem dünnen, Trägermaterial 1 sechs runde Substrate 2 angeordnet sind. Die Substrate 2 bestehen hier aus einer Nutzschicht 2', wie bspw. einer Polymerfolie, falls das Substrat ein Etikett ist, es kann aber auch ein in sich komplizierter aufgebautes therapeutisches Pflaster, mit bspw. Abdeckschicht, Wirkstoffreservoir, ggf. weiteren Stütz- und/oder Steuerschichten und einer Haftklebeschicht 3 sein.

Das Trägermaterial 1 überragt hier die Kontaktflächen der Substrate 2 allseitig. Im Trägermaterial 1 sind in den Kontaktflächen gekrümmte Schnittlinien 4 angeordnet, die die Ränder der Kontaktfläche schneiden, Die hier mit dem Bezugszeichen 5 bezeichneten Anfaßabschnitte des Substrats gleicher oder geringerer Flexibilität als das Trägermaterial werden beim Ablösevorgang durch Druck auf die freie Trägermaterialoberfläche vom Trägermaterial 1 abgehoben und können nun als Anfaßabschnitte 5 dienen.

In Fig. 2 ist ein Querschnitt entlang Linie A-B der Fig. 1 dargestellt. Dabei ist im Trägermaterial 1 die Anordung der Schnitte 4 deutlich zu erkennen, die im Bereich der Kontaktfläche zwischen dem sich aus einer Substratnutzschicht 2' und einer Haftkleberschicht 3 zusammensetzenden Substrat 2 und dem Trägermaterial 1 liegen. Der Schnitt 4 ist so weit vom Substratrand entfernt, daß beim Ablösen ein für das Abziehen des gesamten Substrats 2 hinreichend großer Anfaßabschnitt 5 entsteht.

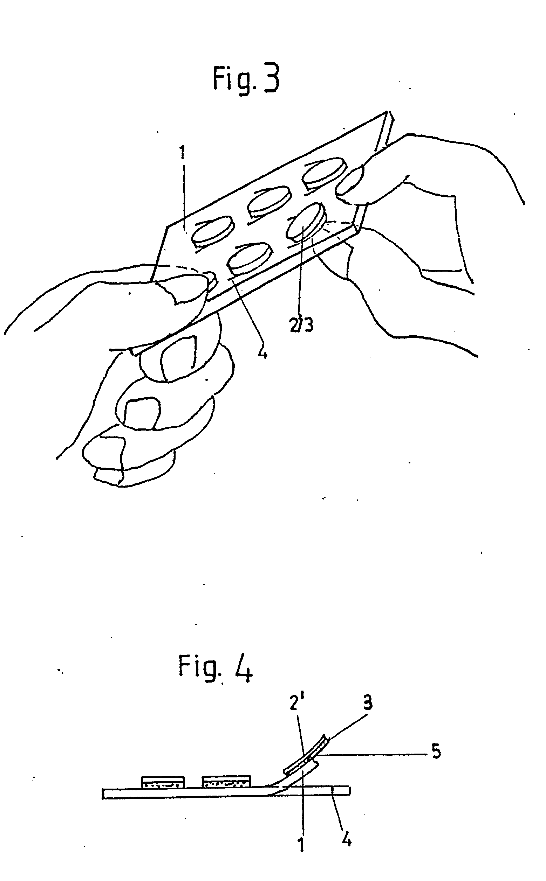

In Fig.3 ist in perspektivischer Darstellung der Einsatz der erfindungsgemäßen Abziehhilfe verdeutlicht, wobei gezeigt ist, wie bei Anwendung von Fingerdruck der Anfaßabschnitt 5 eines Substrats freigelegt werden kann. Das Trägermaterial 1 wird hier mit einer Hand gehalten, während mit dem Daumen der anderen Hand von unten ein Abschnitt der Kontaktfläche des Trägermaterials nach oben gedrückt wird. Dabei löst sich, wie die Querschnittsdarstellung in Fig. 4 zeigt, der Anfaßabschnitt 5 vom Trägermaterial 1 ab. In beiden Fig. sind die Schnitte im Trägermaterial mit dem Bezugszeichen 4 bezeichnet.

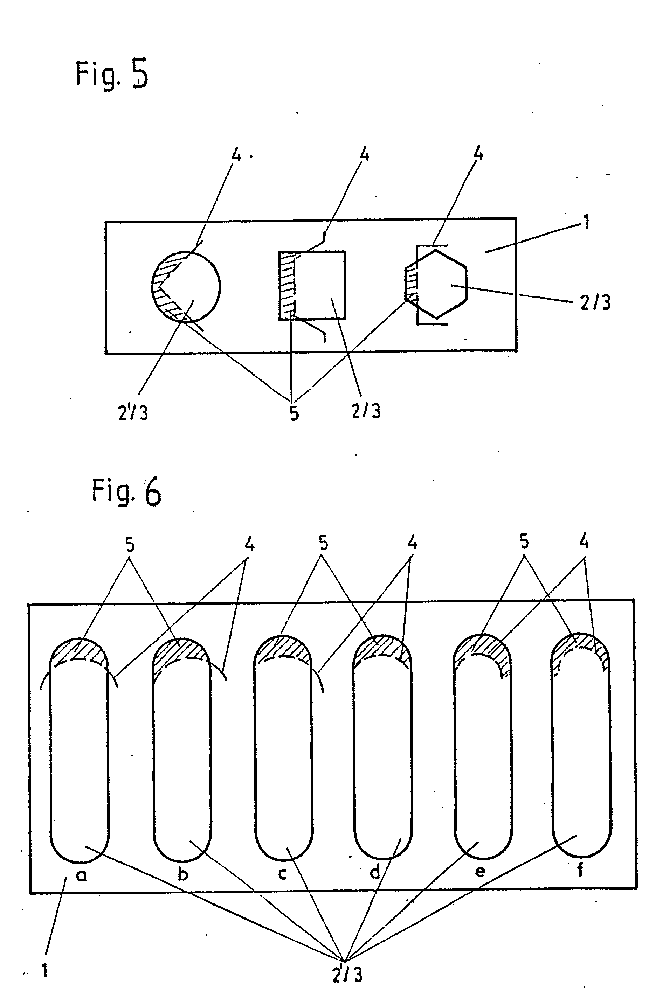

Eine weitere bevorzugte Ausführungsform der Erfindung ist in Fig. 5 dargestellt. Hier haften auf dem Trägermaterial 1 Substrate 2 runder, viereckiger und sechseckiger Form, für deren Ablösung Schnitte 4 in gewinkelter Ausführung vorgesehen sind. So kann der Schnitt zwei Seiten eines Dreiecks, drei Seiten eines Trapezes, drei Seiten eines Vierecks oder auch Teilzüge eines Vielecks darstellen. Die beim Ablösevorgang entstehenden Anfaßabschnitte sind wiederum mit 5 bezeichnet.

In Fig. 6 ist verdeutlicht, daß die Erfindung auch auf Substrate 2 sehr unterschiedlicher Dimensionen anwendbar ist. Es sind 5 Substrate 2 dargestellt, bei denen unterschiedliche mögliche Schnittlinienverläufe vorgesehen sind:

a: die Schnittlinie schneidet den Rand der Kontaktfläche an zwei Seiten;
b: die Schnittlinie berührt einen Kontaktflächenrand und schneidet einen anderen Rand der Kontaktfläche;
c: die Schnittlinie endet einerseits innerhalb und andererseits außerhalb der Kontaktfläche;
d: die Schnittlinie berührt mit ihren Enden zwei Ränder der Kontaktfläche;
e: die Schnittlinie berührt mit einem Ende den Kontaktflächenrand, während das andere Ende innerhalb der Kontaktfläche liegt;
f: beide Schnittlinienenden liegen innerhalb der Kontaktfläche.

In allen diesen Fällen kann durch Druck auf die freie Fläche des Trägermaterials 1 ein ausreichender Anfaßabschnitt 5 des Substrats 2 freigelegt werden.

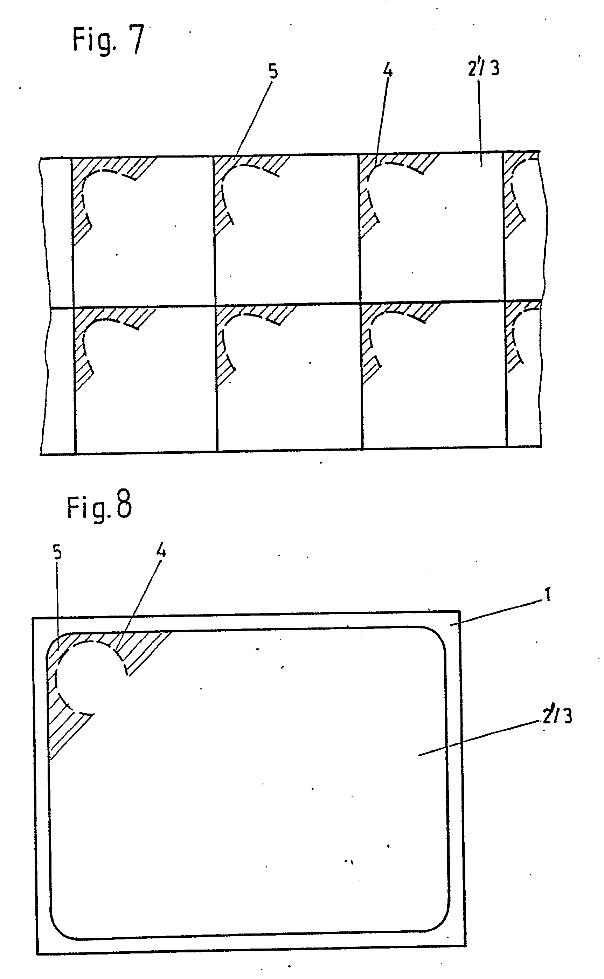

Ein Beispiel für eine weitere bevorzugte Ausführungsform der Erfindung ist in Fig. 7 dargestellt.

Hier ist eine Aufsicht auf einen Abschnitt aus einer bandförmigen Anordnung dargestellt, bei der die durch aneinanderliegende quadratische Substratabschnitte 2, nur durch Schnittlinien voneinander getrennt, aufgeteilte Substratgesamtfläche mit der Fläche des Trägermaterials 1 identisch ist. Durch einen gekrümmten Schnitt 4 im (nicht sichtbaren) Trägermaterial 1 in jeweils einer Ecke eines jeden Substratabschnittes ist die Möglichkeit gegeben, mit Hilfe der durch Druck auf die Unterseite der Anordnung freilegbaren Anfaßabschnitte 5 jedes beliebige Substrat 2 unabhängig von den übrigen Substraten 2 abzuziehen.

Die Fig. 8 schließlich verdeutlicht die Anwendbarkeit der Erfindung auf großformatige Substrate. In der Aufsicht erkennt man das im wesentlichen rechteckige Substrat 2 mit abgerundeten Ecken, das auf einem flächenmäßig etwas größeren Trägermaterial 1 haftet. In einer Ecke der Kontaktfläche ist im Trägermaterial eine gekrümmte Schnittlinie 4 vorgesehen, deren beide Enden innerhalb der Kontaktfläche liegen. Auch hier läßt sich durch Druck auf die Unterseite des Trägermaterials im Bereich des Schnittes der mit 5 bezeichnete Anfaßabschnitt vom Trägermaterial 1 ablösen.

Aus der in der Beschreibung erläuterten und in den Figuren dargestellten Ausführungsform ist leicht zu erkennen, daß sich der Erfindung ein sehr breites Anwendungsgebiet eröffnet und diese erhebliche Vorteile bei der Handhabung von Substraten mit Abdeckmaterialien bietet.

**Patentansprüche**

1. Abziehhilfe für auf einem flächenförmigen flexiblen Trägermaterial haftende, mechanisch ablösbare Substrate in Form von Schnitten oder Sollbruchlinien im Trägermaterial, dadurch gekennzeichnet, daß jeweils nur eine, mindestens teilweise, in der Kontaktfläche des Trägermaterials (1) verlaufende, für jedes Substrat gesonderte, nicht geradlinige Schnitt- oder Sollbruchlinie (4) derart vorgesehen ist, daß bei Anwendung von Druck auf das Trägermaterial (1) mit einer Kraftkomponente in Richtung senkrecht zur Substratkontaktfläche ein durch die Schnitt- oder Sollbruchlinie (4) begrenzter Trägermaterialabschnitt mit auf ihm haftendem Substratabschnitt in Richtung des/der Substrats/e (2) biegbar ist, wodurch mindestens ein Teil des Substrats (2) in dessen Randbereich benachbart der Schnitt-oder Sollbruchlinie (4) vom Trägermaterial (1) abgelöst wird und damit ein Anfaßabschnitt (5) am Substrat zum völligen Abziehen des Substrats (2) gebildet wird.

2. Abziehhilfe nach Anspruch 1, dadurch gekennzeichnet, daß die Fläche des Trägermaterials (1) größer als das/die Substrat/e (2) ist.

3. Abziehhilfe nach einem der Ansprüche 1 oder 2, dadurch gekennzeichnet, daß die Schnitt- oder Sollbruchlinie (4) vollständig innerhalb der Kontaktfläche des Trägermaterials (1) liegt.

4. Abziehhilfe nach einem der Ansprüche 1 oder 2, dadurch gekennzeichnet, daß die Schnitt- bzw. Sollbruchlinie (4) zwei Ränder der Kontaktfläche des Trägermaterials (1) miteinander verbindet.

5. Abziehhilfe nach einem der Ansprüche 1 oder 2, dadurch gekennzeichnet, daß die Schnitt- bzw. Sollbruchlinie (4) mindestens über einen Rand der Kontaktfläche des Trägermaterials hinaus verläuft.

6. Abziehhilfe nach einem der vorangehenden Ansprüche, dadurch gekennzeichnet, daß das Substrat (2) ein flächenförmiges therapeutisches System, ein Etikett od. dgl. ist.

7. Abziehhilfe nach einem der vorangehenden Ansprüche, dadurch gekennzeichnet, daß das Trägermaterial(1) mehrschichtig ist.

8. Abziehhilfe nach einem der vorangehenden Ansprüche, dadurch gekennzeichnet, daß das Substrat (2) mehrschichtig ist.

9. Abziehhilfe nach einem der vorangehenden Ansprüche, dadurch gekennzeichnet, daß die Trägerfolie (1) flexibler als das Substrat (2) ist.

10. Verwendung der Abziehhilfe nach einem der vorangehenden Ansprüche für flächenförmige therapeutische Systeme, Pflaster, Etiketten od. dgl..

## Claims

1. Removal aid for mechanically detachable substrates adhering to a sheet-like, flexible carrier material in the form of cuts or predetermined breaking lines in said carrier material, characterized in that only one separate, non-linear cutting or predetermined breaking line (4) is provided for each substrate at least partially in the contact surface of carrier material (1) in such a way that when pressure is applied to the carrier material (1) with a force component in a direction at right angles to the substrate contact surface, it is possible to bend a carrier material portion bounded by the cutting or predetermined breaking line (4) with the substrate portion adhering to it in the direction of the substrate or substrates (2), so that at least part of substrate (2) is detached in its border area adjacent to the cutting or predetermined breaking line (4) from the carrier material (1) and consequently a gripping portion (5) is formed on the substrate for the complete removal of the latter.

2. Removal aid according to claim 1, characterized in that the surface of the carrier material (1) is larger than the substrate or substrates (2).

3. Removal aid according to one of the claims 1 or 2, characterized in that the cutting or predetermined breaking line (4) is located entirely within the contact surface of the carrier material (1).

4. Removal aid according to one of the claims 1 or 2, characterized in that the cutting or predetermined breaking line (4) interconnects two edges of the contact surface of carrier material (1).

5. Removal aid according to one of the claims 1 or 2, characterized in that the cutting or predetermined breaking line (4) passes beyond at least one edge of the contact surface of the carrier material.

6. Removal aid according to one of the preceding claims, characterized in that the substrate (2) is a sheet-like therapeutic system, a label or the like.

7. Removal aid according to one of the preceding claims, characterized in that the carrier material (1) is in multilayer form.

8. Removal aid according to one of the preceding claims, characterized in that the substrate (2) is in multilayer form.

9. Removal aid according to one of the preceding claims, characterized in that the carrier film (1) is more flexible than the substrate (2).

10. Use of the removal aid according to one of the preceding claims for sheet-like therapeutic systems, plasters, labels, etc.

**Revendications**

1. Dispositif de dégagement auxiliaire pour un substrat mécaniquement pelable adhérant à une matière de support flexible plane sous la forme de découpes ou de lignes de rupture pratiquées dans la matière de support, caractérisé en ce que l'on ménage seulement une ligne de découpe ou de rupture (4) non-rectiligne s'étendant au moins partiellement dans la surface de contact de la matière de support (1) et séparée pour chaque substrat (2) en sorte que, en imprimant une pression sur la matière de support (1) avec une composante de force appliquée perpendiculairement à la surface de contact du substrat, une section de la matière de support limitée par la ligne de découpe ou de rupture (4) et accompagnée de la section du substrat qui y adhère puisse être rabattue dans la direction du/des substrat(s), de manière qu'au moins une partie du substrat (2) puisse être dégagée de la matière de support (1) dans sa zone marginale voisine de la ligne de découpe ou de rupture (4) et que soit ainsi formée une section de prise (5) sur le substrat qui permette de dégager totalement celui-ci.

2. Dispositif de dégagement auxiliaire selon la revendication 1, caractérisé en ce que la surface de la matière de support (1) est plus grande que celle du(des) substrat(s) (2).

3. Dispositif de dégagement auxiliaire selon l'une des revendications 1 et 2, caractérisé en ce que la ligne de découpe ou de rupture (4) se trouve complètement à l'intérieur de la surface de contact de la matière de support (1).

4. Dispositif de dégagement auxiliaire selon l'une des revendications 1 et 2, caractérisé en ce que la ligne de découpe ou de rupture (4) relie l'un à l'autre deux bords de la surface de contact de la matière de support (1).

5. Dispositif de dégagement auxiliaire selon l'une des revendications 1 et 2, caractérisé en ce que la ligne de découpe ou de rupture (4) s'étend vers l'extérieur au moins sur un bord de la surface de contact de la matière de support.

6. Dispositif de dégagement auxiliaire selon l'une des revendications précédentes, caractérisé en ce que le substrat (2) est un système thérapeutique plan, une étiquette ou analogue.

7. Dispositif de dégagement auxiliaire selon l'une des revendications précédentes, caractérisé en ce que la matière de support (1) présente plusieurs couches.

8. Dispositif de dégagement auxiliaire selon l'une des revendications précédentes, caractérisé en ce que le substrat (2) présente plusieurs couches.

9. Dispositif de dégagement auxiliaire selon l'une des revendications précédentes, caractérisé en ce que la feuille de support (1) est plus flexible que le substrat (2).

10. Emploi du dispositif de dégagement auxiliaire selon l'une des revendications précédentes pour des systèmes thérapeutiques plans, pansements, étiquettes ou analogues.

# Fig. 1

# Fig. 2

7

## Fig. 3

## Fig. 4

Fig. 5

Fig. 6

Fig. 7

Fig. 8